(19) Europäisches Patentamt
European Patent Office
Office européen des brevets

(11) EP 4 192 892 B1

(12) EUROPEAN PATENT SPECIFICATION

(45) Date of publication and mention of the grant of the patent:
**24.01.2024 Bulletin 2024/04**

(21) Application number: **22778026.9**

(22) Date of filing: **06.10.2022**

(51) International Patent Classification (IPC):
***C08F 220/20*** *(2006.01)* ***C08L 33/06*** *(2006.01)*
***A61K 8/11*** *(2006.01)* ***C11D 17/00*** *(2006.01)*
***A61K 8/81*** *(2006.01)* ***A61Q 19/00*** *(2006.01)*
***C11D 3/37*** *(2006.01)*

(52) Cooperative Patent Classification (CPC):
**C08F 220/20; A61K 8/11; A61K 8/8152; A61Q 19/00; C08L 33/066; C11D 3/3765; C11D 3/3773; C11D 3/3776;** A61K 2800/10; A61K 2800/48

(86) International application number:
**PCT/EP2022/077810**

(87) International publication number:
**WO 2023/061846 (20.04.2023 Gazette 2023/16)**

# (54) COSMETIC AND DETERGENT COMPOSITION COMPRISING A POLYMERIC COMPOSITION OF A WATER-SOLUBLE POLYMER ENVELOPED WITHIN ANOTHER POLYMER

KOSMETISCHE UND WASCHMITTELZUSAMMENSETZUNG UMFASSEND EINE POLYMERZUSAMMENSETZUNG AUS EINEM POLYMERUMHÜLLTEN WASSERLÖSLICHEN POLYMER

COMPOSITION COSMÉTIQUE ET DÉTERGENTE COMPRENANT UNE COMPOSITION POLYMÈRE D’UN POLYMÈRE HYDROSOLUBLE ENVELOPPÉ DANS UN AUTRE POLYMÈRE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **08.10.2021 FR 2110696**

(43) Date of publication of application:
**14.06.2023 Bulletin 2023/24**

(73) Proprietor: **SNF Group**
**42160 Andrézieux-Bouthéon (FR)**

(72) Inventors:
- **BESSET, Céline**
  **42160 Andrézieux-Bouthéon (FR)**
- **CLEMENT, Benoît**
  **42160 Andrézieux-Bouthéon (FR)**
- **BLONDEL, Frédéric**
  **42160 Andrézieux-Bouthéon (FR)**

(74) Representative: **Cabinet Laurent & Charras**
**Le Contemporain**
**50 Chemin de la Bruyère**
**69574 Dardilly Cedex (FR)**

(56) References cited:
**EP-A1- 1 764 078**
**WO-A1-2019/115619**
**JP-A- H10 251 627**
**US-A1- 2002 012 645**
**US-A1- 2015 044 263**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 192 892 B1

## Description

### TECHNICAL FIELD OF THE INVENTION

The invention relates to a cosmetic or detergent comprising a thickening polymeric composition.

### PRIOR ART

Cosmetic and detergent compositions, such as fabric softeners, present stability problems during their shelf life and/or consumer storage. These instabilities are correlated with an increase in electrolyte content in these compositions over time. These electrolytes are derived from secondary reactions of ingredients in the composition, such as quaternary amine esters. Time and temperature are the main parameters influencing these secondary reactions.

Thickening polymers, such as quaternized di-methylaminoethyl (meth)acrylate ((M)ADAME) based polymers, are known to stabilize cosmetic and detergent compositions. The stabilizing property is provided by hydrated polymers, which can form hydrogels, thickening the aqueous phase of the composition by a charge repulsion mechanism. The range of viscosity obtained depends on the volume developed by the hydrogel, which itself depends on the structure of the polymer and, more precisely, on its cross-linking density.

WO 2019/115619 and FR 3 075 219 relate to a method for preparing a dispersion of a hydrophilic phase comprising a water-soluble polymer and a lipophilic phase. This dispersion is used in an oil recovery method.

US 2002/0012645 discloses a cosmetic composition comprising fluid-encapsulated flexible microspheres, a water-soluble or water-swellable polymer and an aqueous phase.

EP1764078A1 discloses a cosmetic composition containing microcapsules comprising a polymeric and/or waxy envelope, encapsulating an aqueous medium containing an acrylic polymer containing a phosphorylcholine group. The encapsulated aqueous medium may comprise an additional water-soluble polymer, such as carboxymethyl cellulose, xanthan, and guar.

The viscosity obtained by this mechanism shows an increased sensitivity to the presence of electrolytes in the stabilized medium. Electrolytes, generated by the above-mentioned secondary reactions, generally lead to a drop in viscosity and thus in the stability of cosmetic and detergent compositions over time.

Electrolytes are positively or negatively charged chemical substances capable of carrying or conducting an electrical charge.

Currently, to compensate for this loss of stability, manufacturers are over-dosing cosmetic and detergent compositions with thickening polymers so that the impact of the degradation of these polymers is delayed.

The applicant has discovered that encapsulating a thickening polymer in a protective shell made of a certain interfacial or enveloping polymer makes it possible to improve the stability of cosmetic and detergent compositions over time.

More specifically, mixing a first conventional thickening polymer with a second thickening polymer encased (at least partially) in a third polymer forming a protective shell compensates for the drop in viscosity associated with the electrolyte increase in the medium. The integrity of the protective envelope depends on external factors, such as temperature, pH, and shelf life of the cosmetic and detergent compositions. Indeed, the viscosity drop observed over time is compensated by the slow release of the second polymer.

As shown thereafter, the preparation of the polymers is part of a general principle to improve the performance of products and, more particularly, to improve the rheology modifying power. As shown thereafter, the improved performance of the polymers makes it possible to reduce the amount of product needed for the application, which therefore implies a reduction in the release of greenhouse gases such as $CO_2$ associated with the manufacture and use of synthetic polymers.

### Disclosure of the Invention

This invention relates to a cosmetic or detergent composition comprising a thickening polymeric composition. This polymeric composition comprises a dispersion comprising at least one water-soluble polymer A at least partially enveloped by at least one enveloping polymer B. This enveloping polymer B is composed of at least one monomer of formula (I):

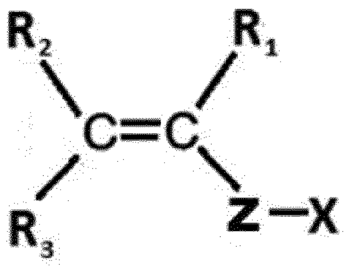

(I)

in which,

* $R_1$, $R_2$, $R_3$ are independently selected from the group consisting of a hydrogen atom, a methyl group, a carboxylate group, and Z-X,
* Z is selected from the group consisting of:

- C(=O)-O;
- C(=O)-O-$R_4$, with $R_4$ = a hydrocarbon chain having from 1 to 20 carbon atoms, for instance (C=O)-O-$CH_2$-CH(OH)-$CH_2$;
- C(=O)-NH;
- C(=O)-NH-$R_4$, with $R_4$ = a hydrocarbon chain having from 1 to 20 carbon atoms;
- O-C(=O);
- O-C(=O) -$R_4$, with $R_4$ = a hydrocarbon chain having from 1 to 20 carbon atoms;
- NH-C(=O)-NH;
- NH-C(=O)-NH-$R_4$, with $R_4$ = a hydrocarbon chain having from 1 to 20 carbon atoms;
- NH-C(=O)-O;
- NH-C(=O)-O-$R_4$, with $R_4$ = a hydrocarbon chain having from 1 to 20 carbon atoms;
- a hydrocarbon chain having from 1 to 20 carbon atoms, for instance $CH_2$;
- a single bond, and

* X is a group selected from alkanolamides, sorbitan esters, ethoxylated sorbitan esters, glyceryl esters, and polyglycosides and wherein X further comprises a hydrocarbon chain having from 2 to 30 carbon atoms.

When Z is and a single bond, the monomer of formula (I) is ($R_2$)($R_3$)C=C($R_1$)(X).

The hydrocarbon chain of Z may or may not be unsaturated. It may be substituted or not, at least partially linear and/or branched and/or comprise at least one cyclic and/or aromatic group. It may comprise one or more heteroatoms advantageously selected from nitrogen and oxygen. The at least one cyclic and/or aromatic group may be on the hydrocarbon chain and/or pendant.

The hydrocarbon chain $R_4$ may or may not be unsaturated. It may be substituted or not, at least partially linear and/or branched and/or comprise at least one cyclic and/or aromatic group. It may comprise one or more heteroatoms advantageously selected from nitrogen and oxygen. The at least one cyclic and/or aromatic group may be on the hydrocarbon chain and/or pendant.

The hydrocarbon chain of X is unsaturated or unsubstituted, substituted or unsubstituted, at least partially linear and/or branched, and/or at least one cyclic and/or aromatic group. It may comprise one or more heteroatoms advantageously selected from nitrogen and oxygen. The at least one cyclic and/or aromatic group may be on the hydrocarbon chain and/or pendant.

Preferably, water-soluble polymer A is completely enveloped by at least one enveloping polymer B. This advantageously makes it possible to transport water-soluble polymer A in a given starting medium virtually without it being in contact with the medium outside the envelope until such time as the external conditions (pH, temperature...) allow water-soluble polymer A to be released into said medium or into a medium which would have replaced the starting medium in the meantime. Once released, water-soluble polymer A can thicken the medium.

The invention relates to a cosmetic or detergent composition comprising said polymeric composition. It also relates to a method to thicken a cosmetic or detergent composition using said polymeric composition. It also relates to a method to maintain the viscosity of a cosmetic or detergent composition over time using said polymeric composition.

The polymeric composition makes it possible to maintain a constant viscosity over time despite an electrolyte increase in the cosmetic or detergent composition. Thus, it is no longer necessary to overdose said cosmetic or detergent composition with a thickening polymer.

The expression "maintain the viscosity over time" means that the viscosity of the thickened cosmetic or detergent

composition that comprises a polymeric composition is maintained at the same value for a period of at least one year, preferably of at least three years. This expression is also used in the case where the viscosity is slightly reduced (by at most 10% of the initial value, preferably by at most 5% of the initial value) but where the loss of viscosity is significantly reduced (by at least 50%, preferably by at least 80%) compared to a thickened cosmetic or detergent composition not comprising the polymeric composition, after a period of one year.

## DESCRIPTION OF THE INVENTION

According to this invention, the term "polymer" refers to a homopolymer or a copolymer, where a copolymer refers to a polymer prepared from at least two different monomers. Therefore it may be a copolymer of at least two monomers selected from anionic monomers, cationic monomers, non-ionic monomers, zwitterionic monomers, and mixtures thereof.

By "water-soluble polymer", we mean a polymer that gives an aqueous solution with no insoluble particles when dissolved under agitation for 4 hours at 25°C and with a concentration of 5 $g.L^{-1}$ in deionized water.

According to the invention, "X and/or Y" means "X" or "Y" or "X and Y".

The invention also includes all possible combinations of the various embodiments disclosed, whether preferred embodiments or those given as an example. In addition, when value ranges are indicated, the terminals are part of these ranges. The disclosure also includes all combinations between the bounds of these value ranges. For example, the value ranges "1-20, preferably 5-15" imply disclosure of the ranges "1-5","1-15", "5-20", and "15-20", and values 1, 5, 15, and 20.

The viscosity is advantageously measured using a Brookfield device. The measurement is carried out at 20°C. The person skilled in the art knows how to adapt the modulus of the Brookfield viscometer and the speed of the modulus according to the viscosity of the fluid whose viscosity he wishes to measure.

### Water-Soluble Polymer A

Water-soluble polymer A may be a natural polymer, a synthetic polymer, or a semi-synthetic polymer. Preferably, water-soluble polymer A is a synthetic polymer.

The term "natural polymer" is meant to be understood as a polymer present in nature, as opposed to a synthetic polymer that is not found in nature, i.e., artificial, even if the starting monomers are natural. Examples of natural polymers are xanthan gums, guar gums, schizophyllan, scleroglucan, or other compounds of the polysaccharide family.

The term "semi-synthetic polymer" is meant to be understood as a natural polymer that has undergone chemical grafting reactions of different synthetic substituents. The person skilled in the art is familiar with such reactions, which are classical chemical reactions applied to natural polymers.

When water-soluble polymer A is a synthetic polymer, it is preferably obtained from at least one monomer selected from the non-ionic monomers, anionic monomers, cationic monomers, zwitterionic monomers, and mixtures thereof. In other words, the water-soluble polymer A can, in particular, be a synthetic polymer obtained from at least one non-ionic monomer and/or at least one anionic monomer and/or at least one cationic monomer and/or a zwitterionic monomer.

The non-ionic monomer or monomers that may be used in the context of the invention may be selected, in particular, from the group comprising water-soluble vinyl monomers. Preferred monomers belonging to this class are, for example, acrylamide, methacrylamide, N-isopropylacrylamide,N,N-dimethylacrylamide, N,N-diethyl acryl amide, N-methylolacrylamide, N-vinylformamide (NVF), N-vinyl acetamide, N-vinylpyridine, N-vinylpyrrolidone (NVP), N-vinyl imidazole, N-vinyl succinimide, acryloyl morpholine (ACMO), acryloyl chloride, glycidyl methacrylate, glyceryl methacrylate, diacetone acrylamide, hydroxyalkyl (meth)acrylate, aminoalkyl (meth)acrylate, thioalkyl (meth)acrylate, and mixtures thereof. Preferably, the non-ionic monomer is an acrylamide.

By (meth)acrylate we mean acrylate or methacrylate.

Generally, the water-soluble polymer advantageously comprises between 0 mol% and 90 mol% of non-ionic monomer, preferably between 5 mol% and 60 mol%, more preferably between 10 mol% and 50 mol%, and even more preferably between 15 mol% and 30 mol%.

The cationic monomer or monomers which may be used in the context of the invention may be selected, in particular, from monomers of the vinyl type, in particular acrylamide, acrylic, allylic, or maleic monomers possessing an ammonium function, preferably quaternary ammonium. We may specifically mention, without limitation, quaternized dimethylaminoethyl acrylate (ADAME), quaternized dimethylaminoethyl methacrylate (MADAME), dimethyldiallylammonium chloride (DADMAC), acrylamido propyltrimethyl ammonium chloride (APTAC), methacrylamido propyltrimethyl ammonium chloride (MAPTAC), and mixtures thereof. Preferably, the cationic monomer is quaternized dimethylaminoethyl acrylate (DMAE).

Generally, the water-soluble polymer advantageously comprises between 10 mol% and 100 mol% of cationic

monomer, preferably between 20 mol% and 95 mol%, more preferably between 30 mol% and 90 mol%, and even more preferably between 40 mol% and 85 mol%.

The person skilled in the art knows how to prepare quaternized monomers, for example, by means of an alkyl halide of the R-X type, R being an alkyl group and X being a halogen, in particular methyl chloride. In addition, it also covers DADMAC, APTAC, and MAPTAC monomers, whose counter-ion is fluoride, bromide, or iodide (instead of chloride).

The anionic monomer(s) being used in the context of the invention may be selected from a broad group. These monomers may have a vinyl function, in particular acrylic, maleic, fumaric, malonic, itaconic, or allylic. They may also contain a carboxylate, phosphonate, phosphate, sulfate, or other anionically charged group. Preferred monomers belonging to this class are, for example, acrylic acid, methacrylic acid, itaconic acid, crotonic acid, maleic acid, fumaric acid, acrylamido undecanoic acid, 3-acrylamido 3-methylbutanoic acid, maleic anhydride, 2-acrylamido-2-methylpropane sulfonic acid (AMPS) vinyl sulfonic acid, vinyl phosphonic acid, allyl sulfonic acid, methallyl sulfonic acid, 2-sulfoethyl methacrylate, sulfopropyl acrylate, methacrylate, allyl phosphonic acid, styrene sulfonic acid, 2-acrylamido-2-methylpropane disulfonic acid, and mixtures thereof. A preferred anionic monomer is 2-acrylamido-2-methylpropane sulfonic acid (AMPS).

Generally, the water-soluble polymer advantageously comprises between 10 mol% and 100 mol% of anionic monomer, preferably between 20 mol% and 95 mol%, more preferably between 30 mol% and 90 mol%, and even more preferably between 40 mol% and 85 mol%.

In one particular embodiment, the anionic monomer(s) may be salified.

By "salified", we mean that at least one acidic function of the anionic monomer is replaced by a salt that neutralizes the negative charge of the acidic function. In other words, the non-salified form corresponds to the acidic form of the monomer, for example, R-C(=O)-OH in the case of the carboxylic acid function, whereas the neutralized form of the monomer corresponds to the R-C(=O)-$O^-X^+$ form, $X^+$ corresponding to a positively charged salt. Neutralization of the acid functions of the water-soluble polymer may be partial or total.

The salified form corresponds advantageously to salts of alkali metals (Li, Na, K...), alkaline earth metals (Ca, Mg...), or ammonium (e.g., ammonium ion or tertiary ammonium). The preferred salts are sodium salts.

Neutralization of the acid function may be partial or total. Generally and advantageously, at least 5% of the anionic monomers, preferably at least 10%, more preferably at least 30%, even more preferably at least 50%, are in salified form.

Salification (formation of the salt) may be done before, during, or after polymerization.

In one particular embodiment, the anionic monomer is derived from a sulfonic acid and is preferably 2-acrylamido-2-methylpropane sulfonic acid in its hydrated form. The hydrated form of 2-acrylamido-2-methylpropane sulfonic acid is one particular form of 2-acrylamido-2-methylpropane sulfonic acid obtainable by controlled crystallization of 2-acrylamido-2-methylpropane sulfonic acid monomer. US 10759746 describes obtaining the hydrated form of 2-acrylamido-2-methylpropane sulfonic acid.

Zwitterionic monomers may also be used in the context of the invention. They combine both anionic and cationic charges on a single monomer. They may be selected, in particular, from monomers of the betaine, sultaine, sulfobetaine, phosphobetaine, carboxybetaine type, and mixtures thereof. Examples of zwitterionic monomers include sulfopropyl dimethylammonium ethyl methacrylate, sulfopropyl dimethylammonium propylmethacrylamide, sulfopropyl 2-vinylpyridinium, and ethyl trimethylammonium ethyl methacrylate phosphate.

The water-soluble polymer may also contain associative monomer(s).

When the water-soluble polymer comprises hydrophobic monomer(s), it generally contains less than 10 mol% thereof.

When the water-soluble polymer comprises hydrophobic monomers, it is present in an amount such that said the water-soluble polymer remains soluble in water.

The polymer may be post-hydrolyzed. Post-hydrolysis is the reaction of a polymer after polymerization. This step consists of the reaction of advantageously non-ionic hydrolyzable functional monomer groups, more advantageously in the reaction of the amide or ester functions with a hydrolysis agent. This hydrolyzing agent may be an enzyme, an ion exchange resin, or an alkali metal. Preferably, the hydrolyzing agent is a Brønsted base. During this post-hydrolysis step of the water-soluble polymer, the number of carboxylic acid functions increases. Indeed, the reaction between the base and the amide or ester functions present in the water-soluble polymer produces carboxylate groups.

The amounts of the various monomer(s) are adjusted by the person skilled in the art so as to not exceed 100% by weight (or 100% molar) when preparing the water-soluble polymer.

In general, the water-soluble polymer does not require the development of a specific polymerization process. Indeed, it may be obtained by following all the polymerization techniques well-known to the person skilled in the art. This may include solution polymerization; gel polymerization; precipitation polymerization; emulsion polymerization (aqueous or inverse); suspension polymerization; reactive extrusion polymerization; water-in-water polymerization; or micellar polymerization.

The water-soluble polymer is preferably obtained by inverse emulsion polymerization.

Generally, the dispersion of the polymeric composition contains between 10% and 60% by weight of the polymer, preferably between 30% and 50% by weight of water-soluble polymer A.

In one preferred embodiment the inverse emulsion comprising the water-soluble polymer is distilled.

The term "distilled" should be understood to mean a partial removal of water from the hydrophilic phase and/or oil from the inverse emulsion. One example of a water removal technique is distillation under reduced pressure. This distillation may be continuous or discontinuous, with azeotropic entrainment. Preferably, the distillation is continuous, and a light oil (i.e., with a boiling point below 200°C) is advantageously used to facilitate the removal of the water.

Generally, the dispersion of the polymeric composition after distillation, contains between 35% and 80% by weight of water-soluble polymer A.

Polymerization is generally a free radical polymerization. According to the invention, "free radical polymerization" should be understood to mean a free radical polymerization by means of at least one initiator (UV, azo, redox, or thermal), or a controlled radical polymerization (CRP) technique, or a matrix polymerization technique.

The water-soluble polymer may have a linear, branched, cross-linked, star-shaped, or comb-shaped structure. This structure may be obtained, according to the general knowledge of the person skilled in the art, for example, by selecting the initiator, the transfer agent, the polymerization technique such as controlled radical polymerization known as RAFT (reversible addition-fragmentation chain transfer),NMP (Nitroxide Mediated Polymerization), or ATRP (Atom Transfer Radical Polymerization), of the incorporation of structural monomers or the concentration.

The water-soluble polymer may further be structured by at least one branching agent, which may be selected from the group consisting of polyethylenically unsaturated monomers (having at least two unsaturated functions) such as for example, vinyl functions, including allyl, acrylic, and epoxy. Examples may include methylene bis acrylamide (MBA), triallyamine, tetraallylammonium chloride, 1,2 dihydroxyethylene bis-(N-acrylamide), and/or macro-initiators such as polyperoxides, polyazoids, and polytransfer agents such as polymercaptans, and polyols.

The water-soluble polymer is preferably structured by methylene bis acrylamide.

Generally speaking, the amount of branching agent is advantageously between 0.005% and 0.1% by weight with respect to the total weight of the monomers constituting the water-soluble polymer. More preferably, it is between 0.01% and 0.095% by weight, even more preferably between 0.03% and 0.08% by weight, and even more preferably between 0.04% and 0.06% by weight. The amount of branching agent is determined by weight of the monomers and is not included in the percentages (molar or mass) of the monomers.

The water-soluble polymer remains soluble in water, even in the presence of a branching agent. The person skilled in the art knows how to adjust the amount of branching agent and possibly the amount of transfer agent, to achieve this result.

Polymerization of the water-soluble polymer may also be conducted with a transfer agent selected from, for example, methanol, isopropyl alcohol, sodium hypophosphite, 2-mercaptoethanol, sodium methallysulfonate, and mixtures thereof. Transfer agents of the xanthate, dithiocarbonate, dithiocarbonate, and trithiocarbonate types and mixtures thereof are also possible. Preferably, the transfer agent is sodium hypophosphite.

Generally, the ratio between the transfer agent and the branching agent is preferably between 100:1 and 1:100, preferably between 50:1 and 1:50, more preferably between 20:1 and 1:20, even more preferably between 10:1 and 1: 10.

The water-soluble polymer A has a molecular weight advantageously between 0.5 and 40 million g/mol, generally at least 1 million g/mol, more preferably between 1 and 30 million g/mol, more preferably between 2 and 20 million g/mol, and even more preferably between 5 and 10 million g/mol. Molecular weight is understood to be the weight-average molecular weight.

Molecular weight may be determined by the intrinsic viscosity of the polymer. Intrinsic viscosity may be measured by any method known to the person skilled in the art. It may be advantageously calculated from the reduced viscosity values for different polymer concentrations by a graphical method consisting of plotting the reduced viscosity values (y-axis) vs. the concentration (x-axis), then extrapolating the curve to zero concentration. The intrinsic viscosity value is plotted on the y-axis or by using the least squares method. The molecular weight may then be determined by the Mark-Houwink equation:

$$[\eta] = K\ M^{\alpha}$$

Where:

$[\eta]$ represents the intrinsic viscosity of the polymer determined by the solution viscosity measurement method,
K represents an empirical constant,
M represents the molecular weight of the polymer,
$\alpha$ represents the Mark-Houwink coefficient, and

K and $\alpha$ depend upon the specific polymer-solvent system.

**Dispersion of Water-Soluble Polymer A**

The dispersion is a dispersion of water-soluble polymer A, whether in a hydrophilic phase or not, in a continuous phase, whether lipophilic or not. The enveloping polymer B is placed at the interface between water-soluble polymer A and the external medium, for example, between a hydrophilic phase containing water-soluble polymer A and a continuous lipophilic phase. Preferably, the hydrophilic phase is an aqueous phase, and the lipophilic phase is an oily phase. Thus, the composition is preferably a water-in-oil dispersion advantageously a water-in-oil emulsion. Water-soluble polymer A enveloped by the enveloping polymer B may also have been in a hydrophilic phase that has been distilled and thus is a "dry" polymer in the envelope formed by the enveloping polymer B.

The enveloping polymer B obtained by polymerization of at least one monomer of formula (I) forms an envelope at the interface between water-soluble polymer A and the external medium, for example, between the hydrophilic phase containing water-soluble polymer A and the continuous, lipophilic, or hydrophilic phase.

As already indicated, preferably, the dispersion is generally in the form of an inverse emulsion.

In one embodiment, the water-soluble polymer A is in the form of micrometric droplets dispersed, advantageously emulsified, in a lipophilic phase. The average size of these droplets is advantageously between 0.01 $\mu$m and 30 $\mu$m, more advantageously between 0.05 $\mu$m and 3 $\mu$m. Enveloping polymer B is therefore placed at the interface between the hydrophilic phase and the lipophilic phase in each droplet. The average droplet size is preferably measured with a laser measuring device using conventional techniques that are part of the person skilled in the art's general knowledge. A Malvern type Mastersizer may be used for this purpose.

Furthermore, in such a case, the dispersion generally has a hydrophilic phase/lipophilic phase weight ratio advantageously between 1 and 100, more advantageously between 5 and 80, and even more advantageously between 10 and 70.

The process for preparing the dispersion is described in the applicant's patent application FR 3 075 219.

**The Enveloping Polymer** B

As already indicated, the enveloping polymer B is obtained from at least one monomer of formula (I):

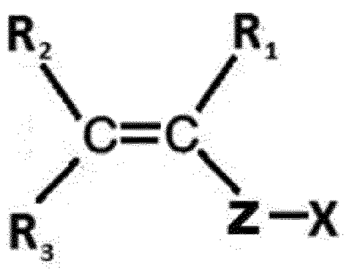

(I)

in which,

*$R_1$, $R_2$, $R_3$ are independently selected from the group consisting of hydrogen, methyl, carboxylate, and Z-X,
*Z is selected from the group consisting of:

- C(=O)-O;
- C(=O)-O-$R_4$, with $R_4$ = a hydrocarbon chain having from 1 to 20 carbon atoms, for instance (C=O)-O-$CH_2$-CH(OH)-$CH_2$;
- C(=O)-NH;
- C(=O)-NH-$R_4$, with $R_4$ = a hydrocarbon chain having from 1 to 20 carbon atoms;
- O-C(=O);
- O-C(=O) -$R_4$, with $R_4$ = a hydrocarbon chain having from 1 to 20 carbon atoms;
- NH-C(=O)-NH;
- NH-C(=O)-NH-$R_4$, with $R_4$ = a hydrocarbon chain having from 1 to 20 carbon atoms;
- NH-C(=O)-O;
- NH-C(=O)-O-$R_4$, with $R_4$ = a hydrocarbon chain having from 1 to 20 carbon atoms;
- a hydrocarbon chain having from 1 to 20 carbon atoms, for instance $CH_2$;
- and a single bond, and

*X is a group selected from alkanolamides, sorbitan esters, ethoxylated sorbitan esters, glyceryl esters, and polyglycosides, and wherein X further comprises a hydrocarbon chain having from 2 to 30 carbon atoms.

The hydrocarbon chain of Z may or may not be unsaturated. It may be substituted or not, at least partially linear and/or branched and/or comprise at least one cyclic and/or aromatic group. It may comprise one or more heteroatoms advantageously selected from nitrogen and oxygen. The at least one cyclic and/or aromatic group may be on the hydrocarbon chain and/or pendant.
The hydrocarbon chain of X is unsaturated or unsubstituted, substituted or unsubstituted, at least partially linear and/or branched, and/or at least one cyclic and/or aromatic group. It may comprise one or more heteroatoms advantageously selected from nitrogen and oxygen. The at least one cyclic and/or aromatic group may be on the hydrocarbon chain and/or pendant.
In other words, X comprises a hydrocarbon chain and a group selected from alkanolamides, sorbitan esters, ethoxylated sorbitan esters glyceryl, and polyglycosides. Advantageously, this hydrocarbon chain comprises from 2 to 30 carbon atoms. According to one preferred embodiment, this hydrocarbon chain is an integral part of the group selected from alkanolamides, sorbitan esters, ethoxylated sorbitan esters, glyceryl esters, and polyglycosides.
Thus, X may be, in particular, one of the following groups:

- an alkanolamide, preferably diethanolamide monooleate (e.g., the commercial compound Witcamide 511), stearoyl ethanolamide (e.g., the commercial compound Witcamide 70), oleic acid monoisopropanolamide (e.g., the commercial compound Witcamide 61), isostearic acid monoisopropanolamide (e.g., the commercial compound Witcamide SPA), coconut monoisopropanolamide (e.g., the commercial compound Empilan® CLS), coco monoethanolamide, oleic acid diethanolamide (e.g., the commercial compound Mexanyl), or oleyl monoisopropanolamide (e.g., the commercial compound Simaline™ IE 101),
- a sorbitan ester, such as, for example, and not exhaustively, sorbitan monolaurate (Span™ 20), sorbitan monopalmitate (Span™ 40), sorbitan monostearate (Span™ 60), sorbitan monoisostearate (Span™ 70), sorbitan tristearate (Span™ 65), sorbitan monooleate (Span™ 80), sorbitan sesquioleate (Span™ 83) or sorbitan trioleate (Span™ 85),
- an ethoxylated sorbitan ester, preferably sorbitan monolaurate polyethylene glycol (Tween™ 20), sorbitan monopalmitate polyethylene glycol (Tween™ 40), sorbitan monostearate polyethylene glycol (Tween™ 60), sorbitan monooleate polyethylene glycol (Tween™ 80) or sorbitan trioleate polyethylene glycol (Tween™ 85),
- a glyceryl ester, preferably polyglycerol monolaurate (Decaglyn 1L), polyglycerol myristate (Decaglyn 1-M), polyglycerol decaoleate (Polyaldo® 10-10-0), polyglycerol distearate (Polyaldo® 6-2-S), polyglycerol oleate (Polyaldo® 10-1-0), polyglycerol caprate (Polyaldo® 10-1 CC KFG), or polyglycerol stearate (Polyaldo® 10-1-S)
- a polyglucoside, preferably decyl glucoside (Triton™ BG-10), lauryl glucoside (Plantacare® 1200UP), capryl glucoside (Plantacare® 810 UP), butyl glucoside (Simulsol™ SL 4), heptyl glucoside (Simulsol™ SL 7 G), octyl and decyl glucoside (Simulsol™ SL 8), decyl glucoside (Simulsol™ SL 10), undecyl glucoside (Simulsol™ SL 11 W), decyl glucoside, and hexadecyl (Simulsol™ SL 26), or octyl hexadecyl glucoside (Simulsol™ SL 826).

According to one particular embodiment, the monomer of formula (I) has an HLB value advantageously lower than 4.5, and advantageously of at least 1.
The HLB value ("hydrophilic-lipophilic balance") makes it possible to calculate the balance existing between the hydrophilic part and the lipophilic part of a molecule. This value is determined by calculating the values of the different parts of the molecule, as described by Griffin in 1949 (Griffin WC, Classification of Surface-Active Agents by HLB, Journal of the Society of Cosmetic Chemists 1 (1949): 311).
In this invention, the conventionally used Griffin method is based on the calculation of the chemical group values of the molecule. Griffin assigned a value between 0 and 20, thus giving information on the solubility in a hydrophilic medium and a lipophilic medium of the molecule. Thus, substances with an HLB of 10 are distributed equally in both phases: their hydrophilic part in the hydrophilic phase and their hydrophobic part in the lipophilic phase.
The formula is:

$$HLB = 20 (Mh/M)$$

Where:

M is the molecular weight of the molecule, and
Mh is the molecular weight of the hydrophilic part.

According to one preferred embodiment, the monomer of formula (I) has the following formula:

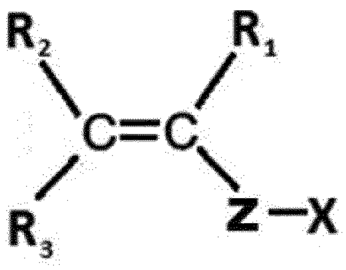

(I)

in which,

* $R_1$, $R_2$, $R_3$ independently are a hydrogen atom or a methyl group,
* Z is selected from the group consisting of $CH_2$, C(=O)-O, C(=O)-NH, and -(C=O)-O-$CH_2$-CH(OH)-$CH_2$,
* X is a group selected from alkanolamides and sorbitan esters comprising a hydrocarbon chain of 2 to 30 carbon atoms.

The hydrocarbon chain of X is unsaturated or unsubstituted, substituted or unsubstituted, at least partially linear and/or branched, and/or at least one cyclic and/or aromatic group. The at least one cyclic and/or aromatic group may be on the hydrocarbon chain and/or pendant.

According to one preferred embodiment, the monomer of formula (1) is selected from sorbitan monooleate (meth)acrylate, 2-hydroxypropyl (meth)acrylate, diethanolamide monooleate, and sorbitan monooleate glyceryl (meth)acrylate.

According to one preferred embodiment, the monomer of formula (I) is the following:

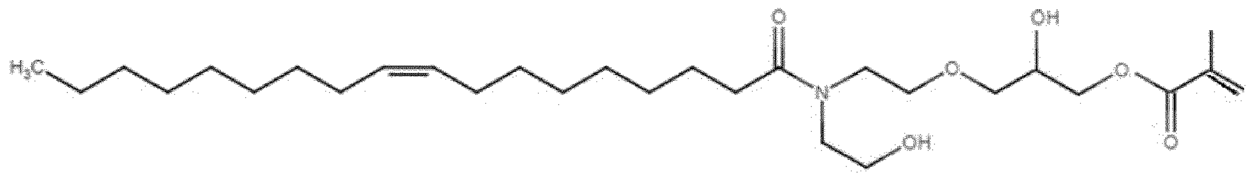

This preferred monomer corresponds to formula $H_3C-(CH_2)_7-CH=CH-(CH_2)_7-C(=O)-N(CH_2CH_2OH)-(CH_2)_2-O-CH_2-CH(OH)-CH_2-O-C(=O)-C(CH_3)=CH_2$.

In one particular embodiment, the enveloping polymer B is obtained by polymerization of at least one monomer of formula (I) and at least one other monomer, preferably at least one monomer selected from non-ionic monomers, anionic monomers, cationic monomers and mixtures thereof. In other words, water-soluble polymer B can specifically be obtained from at least one monomer of formula (I) and at least one other non-ionic monomer and/or at least one anionic monomer and/or at least one cationic monomer.

The monomers used may be selected from the previously cited lists for water-soluble polymer A.

Preferably, the enveloping polymer B comprises between 0.0001% and 10% by weight of monomer of formula (I) based on the total weight of monomers composing the enveloping polymer B, preferably between 0,0001% to 5%, more preferably 0.0001% to 1%, more preferably 0.0001% to 0.5%, even more preferably 0.0001% to 0.1%, and even more preferably 0.0001% to 0.01%.

In general, the time required to degrade (particularly by hydrolysis) the envelope constituted by the enveloping polymer B around water-soluble polymer A increases with the percentage of the monomer of formula (I).

If appropriate, the enveloping polymer B advantageously comprises between 50% and 99.9999%, by weight, of non-ionic monomer relative to the total weight of monomers making up the enveloping polymer B, preferably between 60% and 99.9999% of non-ionic monomer (distinct from the monomer of formula (I)).

Optionally, the enveloping polymer B advantageously comprises between 10% and 99.9999%, by weight, of anionic monomer based on the total weight of monomers component of the enveloping polymer B, preferably between 20% and 99.9999% of non-ionic monomer (distinct from the monomer of formula (I)).

If appropriate, the enveloping polymer B advantageously comprises between 1% and 99.9999%, by weight, of cationic monomer relative to the total weight of monomers making up the enveloping polymer B, preferably between 10% and 99.9999% of non-ionic monomer (distinct from the monomer of formula (I)).

Thus, advantageously, the enveloping polymer B may comprise between 90% and 99.9999% of at least one other non-ionic monomer and/or at least one anionic monomer and/or at least one cationic monomer, preferably between 95% and 99.9999%, more preferably between 99% and 99.9999%, even more preferably between 99.5% and 99.9999%,

more preferably between 99.9% and 99.9999%, even more preferably between 99.99% and 99.9999%, by weight, based on the total weight of monomers making up the enveloping polymer B.

The quantities of the various monomer(s) making up the enveloping polymer B are adjusted by the person skilled in the art so as not to exceed 100% by mass (or 100% molar) during the preparation of the enveloping polymer B.

Advantageously, the enveloping polymer B is neither cross-linked nor branched. It is advantageously linear.

In addition to the above-mentioned monomers, the enveloping polymer B may comprise at least one structural agent. The structural agent is advantageously selected from diacrylamides or methacrylamides of diamines; acrylic esters of di, tri, or tetrahydroxy compounds; methacrylic esters of di, tri, or tetrahydroxy compounds; divinyl compounds preferably separated by an azo group; diallyl compounds preferably separated by an azo group; vinyl esters of di or trifunctional acids; allyl esters of di- or tri-functional acids; methylenebisacrylamide; diallylamine; triallylamine; tetraallylammonium chloride; divinyl sulfone; polyethylene glycol dimethacrylate and diethylene glycol diallyl ether.

**The Envelope**

The enveloping polymer B forms an envelope around water-soluble polymer A, for example, around droplets forming a hydrophilic phase comprising water-soluble polymer A. This envelope is formed during the manufacture of the polymeric composition and is capable, depending on the conditions mainly of pH, temperature, and duration, of losing its envelope function over time by hydrolysis. This allows the polymer to diffuse slowly into the cosmetic or detergent composition. The person skilled in the art is able to determine under what conditions this is possible.

**Polymeric Composition**

In practice, the person skilled in the art has the monomers of water-soluble polymer A and the monomers of the enveloping polymer B, which he or she generally places in the presence of at least one transfer agent and at least one surfactant, in a lipophilic phase, in order to proceed with the simultaneous formation by polymerization of water-soluble polymer A and the enveloping polymer B.

In one preferred embodiment, water-soluble polymer A is prepared, and then enveloping polymer B is formed in the presence of water-soluble polymer A.

According to one embodiment, the polymeric composition may further comprise at least one water-soluble polymer C. Such water-soluble polymer C is as explained below.

**Cosmetic or Detergent Composition**

The invention relates to a cosmetic or detergent composition comprising the preceding polymeric composition, as well as a water-soluble polymer C acting as a thickener.

Water-soluble polymers A and C may be the same or different. Preferably, water-soluble polymers A and C are identical.

The term "different" should be understood to mean that they have at least one different physical characteristic or a different method of preparation, whether, for example, and in a non-exhaustive manner, the monomeric composition, the molecular weight, the presence or absence of a cross-linking agent, the amount of cross-linking agent, the method of polymerization used to prepare the water-soluble polymer, etc.

Water-soluble polymers A and C are different in one particular embodiment.

All that has been stated above with respect to the nature and manufacture of water-soluble polymer A applies identically to water-soluble polymer C.

The polymeric composition may be used according to the knowledge and practices of the formulators of cosmetic and derivative compositions.

In this invention, cosmetic compositions also include dermatological and pharmaceutical compositions. Cosmetic compositions generally comprise an aqueous phase. They are applied in particular to the skin or hair and are generally in the form of oil-in-water emulsions and sometimes water-in-oil emulsions to form creams or lotions, for example. Such compositions may, for example, correspond to anti-aging creams or lotions, aftershaves, moisturizers, hair coloring products, shampoos, conditioners, conditioning shampoos, showering products, cleansing creams, ointments, or sun creams, or lotions. Creams differ from lotions due to their higher viscosity.

Thickeners or rheology modifiers are widely used in these cosmetic products to adapt their sensory profile (appearance, application) to consumer requirements, but also to suspend or stabilize active ingredients. According to the invention, the cosmetic or detergent composition comprises the type of thickener, which is water-soluble polymer C. Water-soluble polymer C may be added to a cosmetic or detergent composition at the same time as the polymeric composition, whether or not separately, or even in a different manner, so as to form the cosmetic or detergent composition according to the invention. It is also possible that a cosmetic or detergent composition comprises a water-soluble polymer

C, and the polymeric composition is added to it so as to form the cosmetic or detergent composition according to the invention.

The term "detergent compositions" is meant to be understood as compositions for cleaning various surfaces, in particular textile fibers, hard surfaces of any kind such as dishes, floors, glass, wood, metal, or composite surfaces. Such compositions correspond, for example, to detergents for washing clothes manually or in a washing machine, products for cleaning dishes manually or for dishwashers, detergents for washing household interiors such as kitchen units, toilets, furniture, floors, windows, and other general-purpose cleaning products.

The amount of the polymeric composition to be added to the cosmetic or detergent composition is generally between 0.01% and 5% by weight, preferably between 0.1% and 3% by weight relative to the weight of the cosmetic or detergent composition.

The cosmetic and detergent compositions may also contain one or more other ingredients well-known to the person skilled in the art, such as water, mineral or vegetable oils, organic solvents, surfactants, cosmetic or pharmaceutical active ingredients, vitamins, chelating agents, emollients, moisturizing agents, botanical extracts, sunscreens, detergent or cleaning additives, fluorescent agents, foaming agents or foam suppressants, neutralizing agents, and, moisturizing agents, botanical extracts, sunscreens, detergent or cleaning additives, fluorescent agents, foaming or foam suppressing agents, neutralizing agents, and pH adjusting agents, preservatives, perfumes, opacifying compounds, dyes, without this list being restrictive. The person skilled in the art knows how and with which ingredients he or she can formulate a cosmetic or detergent composition. As regards cosmetic compositions, reference may be made to application FR 2 979 821. With regard to detergent compositions, reference may be made to documents FR 2 766 838, FR 2 744 131, and EP 0 759 966.

Typically, when the polymeric composition is added to a cosmetic or detergent composition, it makes it possible to manage the loss of viscosity that occurs related to the behavior of water-soluble polymer C over time. Thus, the loss of viscosity is compensated by the release of water-soluble polymer A, which is also thickening, driven by the gradual disappearance of the envelope formed by the enveloping polymer B, as explained below in the description of the figures.

Therefore, another subject-matter relates to a method for thickening a cosmetic or detergent composition using said polymeric composition.

Lastly, another subject-matter relates to a method for maintaining the viscosity of a cosmetic or detergent composition over time using said polymeric composition.

## DESCRIPTION OF THE FIGURES

Figure 1 is a schematic illustration of an example of a polymeric composition for the invention, which is a dispersion. It shows a water-soluble polymer A made up of microdroplets, each trapped in an envelope made of an enveloping polymer B. In this case, water-soluble polymer A may be in a hydrophilic phase forming droplets in a continuous lipophilic phase, the enveloping polymer B being at the interface between the hydrophilic phase and the lipophilic phase. Water-soluble polymer A may also be in solid form, forming a dispersion in a lipophilic or hydrophilic continuous phase, with the enveloping polymer B at the interface between water-soluble polymer A (solid form) and the continuous phase (hydrophilic or lipophilic).

Figure 2 is a schematic illustration of a cosmetic or detergent composition comprising a polymeric composition for the invention, as shown in Figure 1, shortly after the polymeric composition has been added to a cosmetic or detergent composition in the presence of a water-soluble polymer C. In this case, water-soluble polymer A may be in solid form or in a hydrophilic phase (forming droplets) in a continuous phase (hydrophilic or lipophilic), the enveloping polymer B being at the interface between water-soluble polymer A (solid or in a hydrophilic phase) and the continuous phase (lipophilic or hydrophilic) of the cosmetic composition.

Figure 3 is a schematic illustration of the cosmetic or detergent composition of Figure 2 after a certain time, during which the phenomenon of viscosity loss over time has occurred. The increase in electrolytes (e-) led to less ionic repulsion between water-soluble C polymer chains, which caused a drop in viscosity.

Figure 4 is a schematic illustration of the cosmetic or detergent composition of Figure 3 after more time has passed, during which the degradation (at least in part) of part of the protective envelopes of water-soluble polymers A has occurred (with time, temperature, and/or pH), leading to the slow liberation of part of water-soluble polymers A, thus compensating for the loss of viscosity of the cosmetic or detergent composition over time.

Figure 5 is a schematic illustration of the cosmetic or detergent composition of Figure 4 after even more time has

passed, during which the degradation of the protective envelopes of water-soluble polymers A continued, leading to the release of more and more water-soluble polymer A and during the same time a volume setting of the clusters of water-soluble polymer A and thus maintenance of the viscosity of the cosmetic or detergent composition over time.

## Claims

1. A cosmetic or detergent composition comprising a polymeric composition, for thickening said cosmetic or detergent composition,

   said polymeric composition comprising a dispersion comprising at least one water-soluble polymer A at least partially enveloped by at least one enveloping polymer B,
   the enveloping polymer B being composed of at least one monomer of formula (I):

   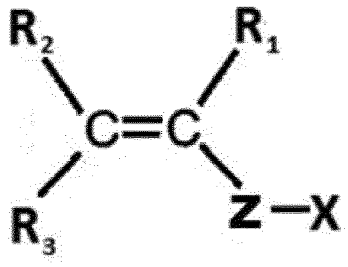

   (I)

   wherein,

   * $R_1$, $R_2$, $R_3$ are independently selected from the group consisting of a hydrogen atom, a methyl group, a carboxylate group, and Z-X,
   * Z is selected from the group consisting of:

     - C(=O)-O;
     - C(=O)-O-$R_4$, with $R_4$ = a hydrocarbon chain having from 1 to 20 carbon atoms;
     - C(=O)-NH;
     - C(=O)-NH-$R_4$, with $R_4$ = a hydrocarbon chain having from 1 to 20 carbon atoms;
     - O-C(=O);
     - O-C(=O) -$R_4$, with $R_4$ = a hydrocarbon chain having from 1 to 20 carbon atoms;
     - NH-C(=O)-NH;
     - NH-C(=O)-NH-$R_4$, with $R_4$ = a hydrocarbon chain having from 1 to 20 carbon atoms;
     - NH-C(=O)-O;
     - NH-C(=O)-O-$R_4$, with $R_4$ = a hydrocarbon chain having from 1 to 20 carbon atoms;
     - a hydrocarbon chain having from 1 to 20 carbon atoms;
     - and a single bond, and

   * X is a group selected from alkanolamides, sorbitan esters, ethoxylated sorbitan esters, glyceryl esters, and polyglycosides; and wherein X further comprises a hydrocarbon chain having from 2 to 30 carbon atoms.

2. The composition according to claim 1 **characterized in that** water-soluble polymer A is a synthetic polymer.

3. The composition according to either of claims 1 or 2, **characterized in that** water-soluble polymer A is a synthetic polymer obtained from at least one non-ionic monomer and/or at least one anionic monomer and/or at least one cationic monomer and/or one zwitterionic monomer.

4. The composition according to claim 3, **characterized in that** the non-ionic monomer or monomers are selected from acrylamide, methacrylamide, N-isopropylacrylamide, N,N-dimethylacrylamide, N,N-diethylacrylamide, N-methylolacrylamide, N-vinylformamide (NVF), N-vinyl acetamide, N-vinylpyridine, and N-vinylpyrrolidone (NVP), N-vinyl imidazole, N-vinyl succinimide, acryloyl morpholine (ACMO), acryloyl chloride, glycidyl methacrylate, glyceryl methacrylate, diacetone acrylamide, hydroxyalkyl (meth)acrylate, aminoalkyl (meth)acrylate, thioalkyl (meth)acrylate, and mixtures thereof.

**5.** The composition according to claim 3, **characterized in that** the cationic monomer or monomers are selected from vinyl type monomers, in particular, acrylamide, acrylic, allylic, or maleic monomers possessing an ammonium function, advantageously quaternary ammonium.

**6.** The composition according to claim 3, **characterized in that** the anionic monomer or monomers are selected from monomers having a vinyl function, in particular acrylic, maleic, fumaric, malonic, itaconic, or allylic.

**7.** The composition according to any of the preceding claims, **characterized in that** water-soluble polymer A is structured by at least one branching agent.

**8.** The composition according to any of the preceding claims, **characterized in that** water-soluble polymer A has a weight-average molecular weight between 0.5 and 40 million g/mol, measured by the method disclosed in the description.

**9.** The composition according to any of the preceding claims, **characterized in that** the enveloping polymer B is obtained by polymerization of at least one monomer of formula (I) and at least one other monomer, preferably at least one monomer selected from non-ionic monomers, anionic monomers, cationic monomers and mixtures thereof.

**10.** Composition according to any of the preceding claims, **characterized in that** the monomer of formula (I) has the following formula:

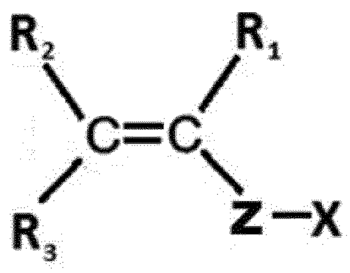

(I)

wherein,

* $R_1$, $R_2$, $R_3$ are independently an hydrogen atom or a methyl group,
* Z is selected from the group consisting of $CH_2$, C(=O)-O, C(=O)-NH, and -(C=O)-O-CHz-CH(OH)-$CH_2$,
* X is a group selected from alkanolamides and sorbitan esters, comprising a hydrocarbon chain of 2 to 30 carbon atoms.

**11.** The composition according to any of the preceding claims, **characterized in that** the monomer of formula (I) is selected from sorbitan monooleate (meth)acrylate, diethanolamide monooleate 2-hydroxypropyl (meth)acrylate, and sorbitan monooleate glyceryl (meth)acrylate.

**12.** The composition according to any of the preceding claims, **characterized in that** the monomer of formula (I) is the following:

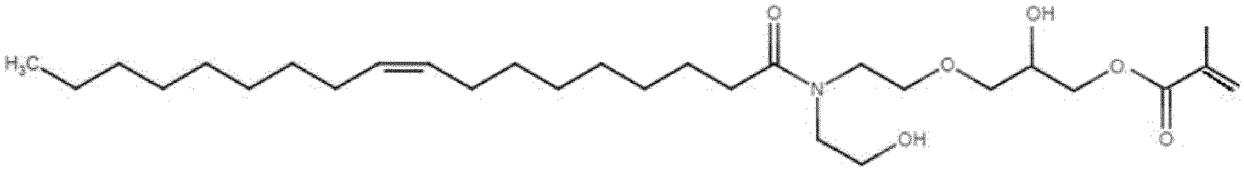

**13.** The composition according to any of the preceding claims, **characterized in that** it further comprises at least one water-soluble polymer C.

**14.** A method for thickening a cosmetic or detergent composition using a polymeric composition comprising a dispersion comprising at least one water-soluble polymer A at least partially enveloped by at least one enveloping polymer B, the enveloping polymer B being composed of at least one monomer of formula (I):

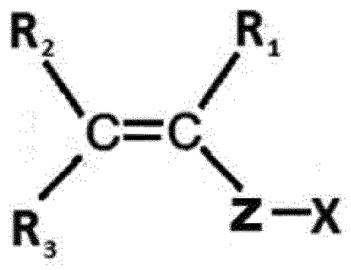

(I)

wherein,

* $R_1$, $R_2$, $R_3$ are independently selected from the group consisting of a hydrogen atom, a methyl group, a carboxylate group, and Z-X,
* Z is selected from the group consisting of:

- C(=O)-O;
- C(=O)-O-$R_4$, with $R_4$ = a hydrocarbon chain having from 1 to 20 carbon atoms;
- C(=O)-NH;
- C(=O)-NH-$R_4$, with $R_4$ = a hydrocarbon chain having from 1 to 20 carbon atoms;
- O-C(=O);
- O-C(=O) -$R_4$, with $R_4$ = a hydrocarbon chain having from 1 to 20 carbon atoms;
- NH-C(=O)-NH;
- NH-C(=O)-NH-$R_4$, with $R_4$ = a hydrocarbon chain having from 1 to 20 carbon atoms;
- NH-C(=O)-O;
- NH-C(=O)-O-$R_4$, with $R_4$ = a hydrocarbon chain having from 1 to 20 carbon atoms;
- a hydrocarbon chain having from 1 to 20 carbon atoms;
- and a single bond, and

* X is a group selected from alkanolamides, sorbitan esters, ethoxylated sorbitan esters, glyceryl esters, and polyglycosides; and wherein X further comprises a hydrocarbon chain having from 2 to 30 carbon atoms.

**Patentansprüche**

**1.** Eine kosmetische oder Waschmittelzusammensetzung umfassend eine Polymerzusammensetzung zur Verdickung dieser kosmetischen oder Waschmittelzusammensetzung,

diese Polymerzusammensetzung enthält eine Dispersion umfassend mindestens ein wasserlösliches Polymer A, zumindest zum Teil eingehüllt von mindestens einem umhüllenden Polymer B,
das umhüllende Polymer B setzt sich dabei zusammen aus mindestens einem Monomer mit der Formel (I):

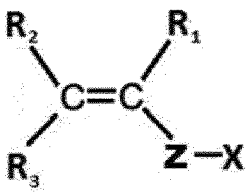

(I)

(I)

Worin,

* $R_1$, $R_2$, $R_3$ unabhängig voneinander ausgewählt werden aus der Gruppe bestehend aus einem Wasserstoffatom, einer Methylgruppe, einer Carboxylatgruppe, und Z-X,
* Z wird dabei ausgewählt wird aus der Gruppe bestehend aus:

- C(=0)-0;
- C(=0)-0-R4, mit R4 = eine Kohlenwasserstoffkette, mit 1 bis 20 Kohlenstoffatomen;
- C(=0)-NH;
- C(=0)-NH-R.4, mit R4 = eine Kohlenwasserstoffkette, mit 1 bis 20 Kohlenstoffatomen;
- 0-C(=0);
- 0-C(=0) -R4, mit R4 = eine Kohlenwasserstoffkette, mit 1 bis 20 Kohlenstoffatomen;
- NH-C(=0)-NH;
- NH-C(=0)-NH-R4, mit R4 = eine Kohlenwasserstoffkette, mit 1 bis 20 Kohlenstoffatomen;
- NH-C(=0)-0;
- NH- C(=0)-0-R4, mit R4 = eine Kohlenwasserstoffkette, mit 1 bis 20 Kohlenstoffatomen;
- eine Kohlenwasserstoffkette, mit 1 bis 20 Kohlenstoffatomen;
- und eine einfache Bindung, und

* X ist eine Gruppe, ausgewählt aus den Alkanolamiden, Sorbitanestern, ethoxylierten Sorbitanestern, Glycerinfettsäureestern und Polyglycosiden; und worin X weiterhin eine Kohlenwasserstoffkette mit 2 bis 30 Kohlenstoffatomen enthält.

2. Die Zusammensetzung gemäß Anspruch 1, **dadurch gekennzeichnet, dass** das wasserlösliche Polymer A ein synthetisches Polymer ist.

3. Zusammensetzung nach Anspruch 1 oder 2, **dadurch gekennzeichnet, dass** das wasserlösliche Polymer ein synthetisches Polymer ist, erhalten durch Polymerisation mindestens eines nicht-ionischen Monomers und/ oder mindestens eines anionischen Monomers und/ oder mindestens eines anionischen Monomers und/ oder mindestens eines kationisches Monomer und/ oder eines zwitterionischen Monomers.

4. Zusammensetzung nach Anspruch 3, **dadurch gekennzeichnet, dass** das oder die nicht-ionische(n) Monomere ausgewählt wird(werden) aus Acrylamid, Methacrylamid, N-isopropylacrylamid, N,N- dimethylacrylamid, N,N-di ethyl acrylamid, N-methylolacrylamid, N-vinylformamid (NVF), N-vinylacetamid, N-vinylpyridin, und N-vinylpyrrolidon (NVP), N-vinylimidazol, N-vinylsuccinimid, Acryloylmorpholin (ACMO), Acryloylchlorid, Glycidylmethacrylat, Glycerylmethacrylat, Diacetonacrylamid, Hydroxyalkyl (meth)acrylat, Aminoalkyl (meth)acrylat, Thioalkyl (meth)acrylat, und Mischungen daraus.

5. Zusammensetzung nach Anspruch 3, **dadurch gekennzeichnet, dass** das oder die kationische(n) Monomere ausgewählt wird(werden) aus Monomeren vom Vinyltyp, insbesondere Acrylamid-, Acryl-, Allyl-, oder Maleinmonomere, die eine Ammoniumfunktion enthalten, vorzugsweise quaternäres Ammonium.

6. Zusammensetzung nach Anspruch 3, **dadurch gekennzeichnet, dass** das oder die anionische(n) Monomere ausgewählt wird(werden) aus eine Vinylfunktion, insbesondere Acryl, Malein, Fumar, Malon, Itacon, oder Allyl.

7. Zusammensetzung nach irgendeinem der vorangehenden Ansprüche, **dadurch gekennzeichnet, dass** das das wasserlösliche Polymer A durch mindestens einen Verzweiger strukturiert wird.

8. Zusammensetzung nach irgendeinem der vorangehenden Ansprüche, **dadurch gekennzeichnet, dass** das wasserlösliche Polymer A ein Gewichtsmittel-Molekulargewicht von zwischen 0.5 und 40 Millionen g/mol, gemessen mit dem in der Beschreibung offengelegten Verfahren, hat.

9. Zusammensetzung nach irgendeinem der vorangehenden Ansprüche, **dadurch gekennzeichnet, dass** das umhüllende Polymer B erhalten wird durch Polymerisation mindestens eines Monomers mit der Formel (I) und mindestens eines weiteren Monomers, vorzugsweise mindestens ein Monomer ausgewählt aus nicht-ionischen Monomeren, anionischen Monomeren, kationischen Monomeren und Mischungen daraus.

10. Zusammensetzung nach irgendeinem der vorangehenden Ansprüche, **dadurch gekennzeichnet, dass** das Monomer mit der Formel (I) die folgende Formel hat:

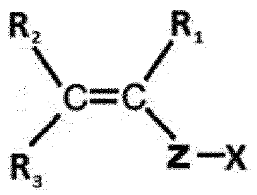

(I)

worin,

* $R_1$, $R_2$, $R_3$ unabhängig voneinander ein Wasserstoffatom oder eine Methylgruppe sind,
* Z ausgewählt wird aus der Gruppe bestehend aus CH2, C(=0)-0, C(=0)-NH, und - (C=0)-0-CH2- CH(OH)-$CH_2$,
* X eine Gruppe ist ausgewählt aus Alkanolamiden und Sorbitanestern, umfassend eine Kohlenwasserstoffkette von 2 bis 30 Kohlenstoffatomen.

**11.** Zusammensetzung nach irgendeinem der vorangehenden Ansprüche, **dadurch gekennzeichnet, dass** das Monomer der Formel (I) ausgewählt wird aus Sorbitanmonooleat (meth)acrylat, Di-ethanol amid- monooleat 2-hydroxypropyl (meth)acrylat, und Sorbitan monooleat glyceryl (meth)acrylat.

**12.** Zusammensetzung nach irgendeinem der vorangehenden Ansprüche, **dadurch gekennzeichnet, dass** das Monomer mit der Formel (I) das folgende ist:

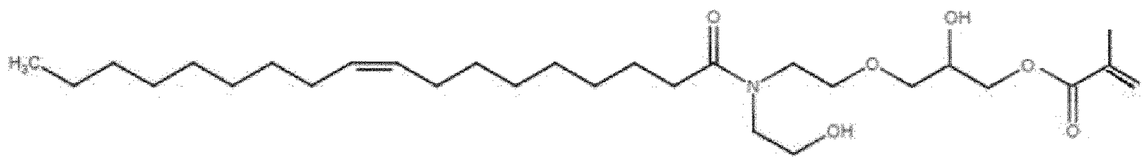

**13.** Zusammensetzung nach irgendeinem der vorangehenden Ansprüche, **dadurch gekennzeichnet, dass** sie außerdem mindestens ein wasserlösliches Polymer C enthält.

**14.** Verfahren zur Verdickung einer kosmetischen oder Waschmittelzusammensetzung unter Verwendung einer Polymerzusammensetzung umfassend eine Dispersion umfassend mindestens ein wasserlösliches Polymer A zumindest zum Teil eingehüllt von mindestens einem umhüllenden Polymer B, das umhüllende Polymer B setzt sich dabei zusammen aus mindestens einem Monomer mit der Formel (I):

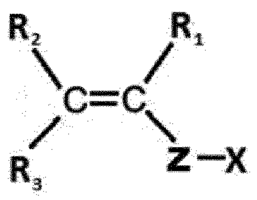

(I)

worin,

$R_1$, $R_2$, $R_3$ unabhängig ausgewählt werden aus der Gruppe bestehend aus einem Wasserstoffatom, einer Methylgruppe, einer Carboxylatgruppe, und Z-X,
* Z wird dabei ausgewählt wird aus der Gruppe bestehend aus:

- C(=0)-0;
- C(=0)-0-R4, mit R4 = eine Kohlenwasserstoffkette, mit 1 bis 20 Kohlenstoffatomen;
- C(=0)-NH;
- C(=0)-NH-R4, mit R4 = eine Kohlenwasserstoffkette, mit 1 bis 20 Kohlenstoffatomen;
- 0-C(=0);
- 0-C(=0)-R4, mit R4 = eine Kohlenwasserstoffkette, mit 1 bis 20 Kohlenstoffatomen;
- NH-C(=0)-NH;
- NH-C(=0)-NH-R4, mit R4 = eine Kohlenwasserstoffkette mit 1 bis 20 Kohlenstoffatomen;

- NH-C(=0)-0;
- NH-C (=0)-0-R4, mit R4 =eine Kohlenwasserstoffkette, mit 1 bis 20 Kohlenstoffatomen;
- eine Kohlenwasserstoffkette, mit 1 bis 20 Kohlenstoffatomen;
- und eine einfache Bindung, und

* X ist eine Gruppe, ausgewählt aus Alkanolamiden, Sorbitanestern, ethoxylierten Sorbitanestern, Glycerinfettsäureester, und Polyglycosiden; und worin X weiterhin eine Kohlenwasserstoffkette mit 2 bis 30 Kohlenstoffatomen enthält.

## Revendications

1. Composition cosmétique ou détergente comprenant une composition polymérique pour épaissir ladite composition cosmétique ou détergente, ladite composition polymérique comprenant une dispersion comprenant au moins un polymère hydrosoluble A au moins partiellement enveloppé par au moins un polymère enveloppant B,
le polymère enveloppant B étant composé d'au moins un monomère de formule (I) :

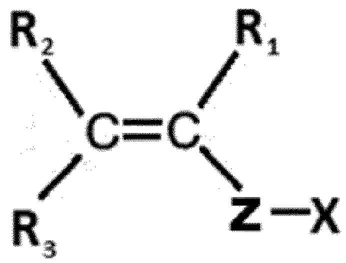

(I)

dans laquelle,

* $R_1$, $R_2$, $R_3$ sont indépendamment choisis dans le groupe comprenant un atome d'hydrogène, un groupe méthyle, un groupement carboxylate et Z-X,
* Z est choisi dans le groupe consistant en :

- C(=O)-O ;
- C(=O)-O-R4, avec R4 = chaine hydrocarbonée ayant de 1 à 20 atomes de carbone;
- C(=O)-NH ;
- C(=O)-NH-R4, avec R4 = chaine hydrocarbonée ayant de 1 à 20 atomes de carbone;
- O-C(=O) ;
- C(=O)-R4, avec R4 = chaine hydrocarbonée ayant de 1 à 20 atomes de carbone;
- NH-C(=O)-NH ;
- NH-C(=O)-NH-R4, avec R4 = chaine hydrocarbonée ayant de 1 à 20 atomes de carbone
- NH-C(=O)-O
- NH-C(=O)-O-R4 , avec R4 = chaine hydrocarbonée ayant de 1 à 20 atomes de carbone
- une chaine hydrocarbonée comportant de 1 à 20 atomes de carbone, par exemple CH2,
- une simple liaison et

* X est un groupe choisi parmi les alcanolamides, les esters de sorbitane, les esters de sorbitane éthoxylés, les esters de glycéryle, et les polyglycosides ; et dans lequel X comprenant en outre une chaine hydrocarbonée comportant de 2 à 30 atomes de carbone.

2. Composition selon la revendication 1, **caractérisée en ce que** le polymère hydrosoluble A est un polymère synthétique.

3. Composition selon l'une des revendication 1 ou 2, **caractérisée en ce que** le polymère hydrosoluble A est un polymère synthétique obtenu à partir d'au moins un monomère non ionique et/ou au moins un monomère anionique et/ou au moins un monomère cationique et/ou un monomère zwittérionique.

4. Composition selon la revendication 3, **caractérisée en ce que** le ou les monomères non ioniques sont choisis parmi

l'acrylamide, le méthacrylamide, le N-isopropylacrylamide, le N,N-diméthylacrylamide, le N,N-diéthylacrylamide le N-méthylolacrylamide, la N-vinylformamide (NVF), le N-vinyl acétamide, la N-vinylpyridine et la N-vinylpyrrolidone (NVP), le N-vinyl imidazole, le N-vinyl succinimide, l'acryloyl morpholine (ACMO), le chlorure d'acryloyl, le méthacrylate de glycidyle, le méthacrylate de glycéryle, la diacétone acrylamide, les hydroxyalkyl (méth)acrylate, les aminoalkyl (méth)acrylate, les thioalkyl (méth)acrylate et leurs mélanges.

5. Composition selon la revendication 3, **caractérisée en ce que** le ou les monomères cationiques sont choisis, parmi les monomères du type vinylique, notamment acrylamide, acrylique, allylique ou maléique possédant une fonction ammonium, avantageusement ammonium quaternaire.

6. Composition selon la revendication 3, **caractérisée en ce que** le ou les monomères anioniques sont choisis parmi les monomères présentant une fonction vinylique notamment acrylique, maléique, fumarique, malonique, itaconique, ou allylique.

7. Composition selon l'une des revendication précédentes, **caractérisée en ce que** le polymère hydrosoluble A est structuré par au moins un agent ramifiant.

8. Composition selon l'une des revendication précédentes, **caractérisée en ce que** le polymère hydrosoluble A a un poids moléculaire compris entre 0,5 et 40 million g/mol mesurée selon la méthode mentionnée dans la description.

9. Composition selon l'une des revendication précédentes, **caractérisée en ce que** le polymère hydrosoluble C est obtenu par polymérisation d'au moins un monomère de formule (I) et d'au moins autre monomère, de préférence un monomère non ionique et/ou au moins un monomère anionique et/ou au moins un monomère cationique.

10. Composition selon l'une des revendication précédentes, **caractérisée en ce que** le monomère de formule (I) a la formule suivante :

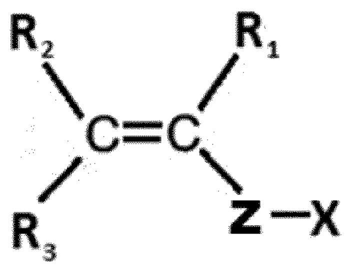

(I)

dans laquelle,

- * $R_1$, $R_2$, $R_3$ indépendamment sont un atome d'hydrogène ou un groupe méthyle,
- * Z est choisi dans le groupe comprenant $CH_2$, C(=O)-O, C(=O)-NH, et -(C=O)-O-$CH_2$-CH(OH)-$CH_2$,
- * X est un groupe choisi parmi les alcanolamides et les esters de sorbitane, et comprenant une chaine hydrocarbonéecomportant de 2 à 30 atomes de carbone.

11. Composition selon l'une des revendication précédentes, **caractérisée en ce que** le monomère de formule (I) est choisi parmi le (méth)acrylate du monooléate de sorbitane, le (méth)acrylate de 2-hydroxypropyl du monooléate de diéthanolamide et le (méth)acrylate glycéryl du monooléate de sorbitane.

12. Composition selon l'une des revendication précédentes, **caractérisée en ce que** le monomère de formule (I) est le suivant :

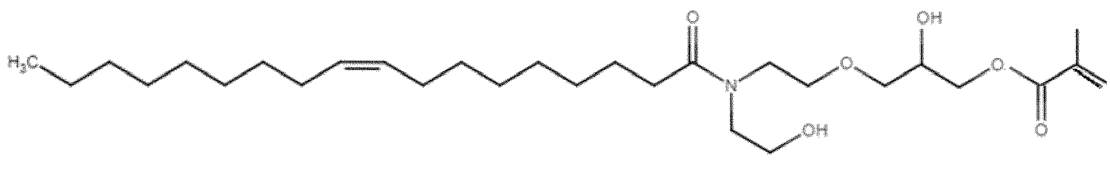

13. Composition selon l'une des revendications précédentes, **caractérisée en ce qu'**elle comprend en outre au moins

un polymère hydrosoluble C.

**14.** Méthode pour épaissir une composition cosmétique ou détergente en utilisant la composition polymérique comprenant une dispersion comprenant au moins un polymère A hydrosoluble au moins partiellement enveloppé par au moins un polymère B enveloppant, le polymère B enveloppant étant composé d'au moins un monomère de formule I

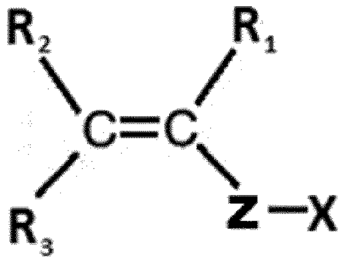

(I)

dans laquelle,

* $R_1$, $R_2$, $R_3$ sont indépendamment choisis dans le groupe comprenant un atome d'hydrogène, un groupe méthyle, un groupement carboxylate et Z-X,
* Z est choisi dans le groupe consistant en :

- C(=O)-O ;
- C(=O)-O-R4, avec R4 = chaine hydrocarbonée ayant de 1 à 20 atomes de carbone;
- C(=O)-NH ;
- C(=O)-NH-R4, avec R4 = chaine hydrocarbonée ayant de 1 à 20 atomes de carbone;
- O-C(=O) ;
- C(=O)-R4, avec R4 = chaine hydrocarbonée ayant de 1 à 20 atomes de carbone;
- NH-C(=O)-NH ;
- NH-C(=O)-NH-R4, avec R4 = chaine hydrocarbonée ayant de 1 à 20 atomes de carbone
- NH-C(=O)-O
- NH-C(=O)-O-R4 , avec R4 = chaine hydrocarbonée ayant de 1 à 20 atomes de carbone
- une chaine hydrocarbonée comportant de 1 à 20 atomes de carbone, par exemple CH2,
- une simple liaison et

* X est un groupe choisi parmi les alcanolamides, les esters de sorbitane, les esters de sorbitane éthoxylés, les esters de glycéryle, et les polyglycosides ; et dans lequel X comprenant en outre une chaine hydrocarbonée comportant de 2 à 30 atomes de carbone.

[Fig. 1]

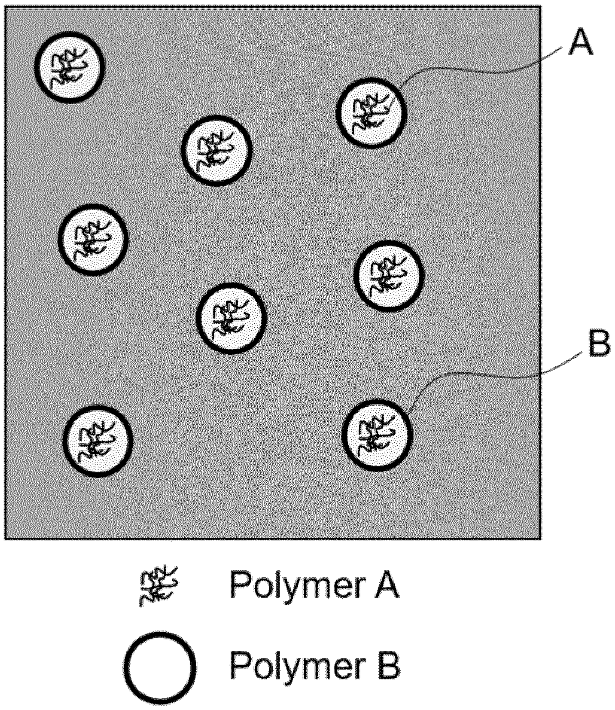


[Fig. 2]

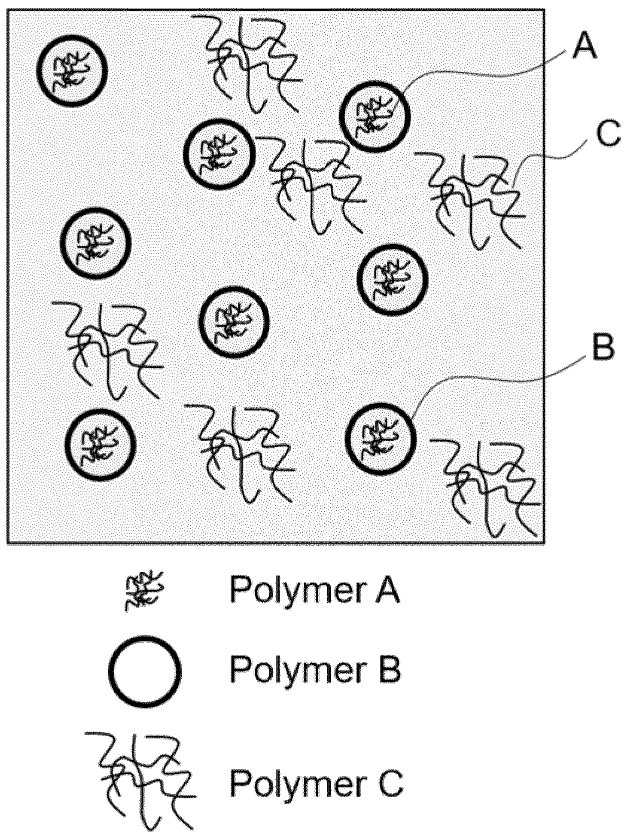

[Fig. 3]

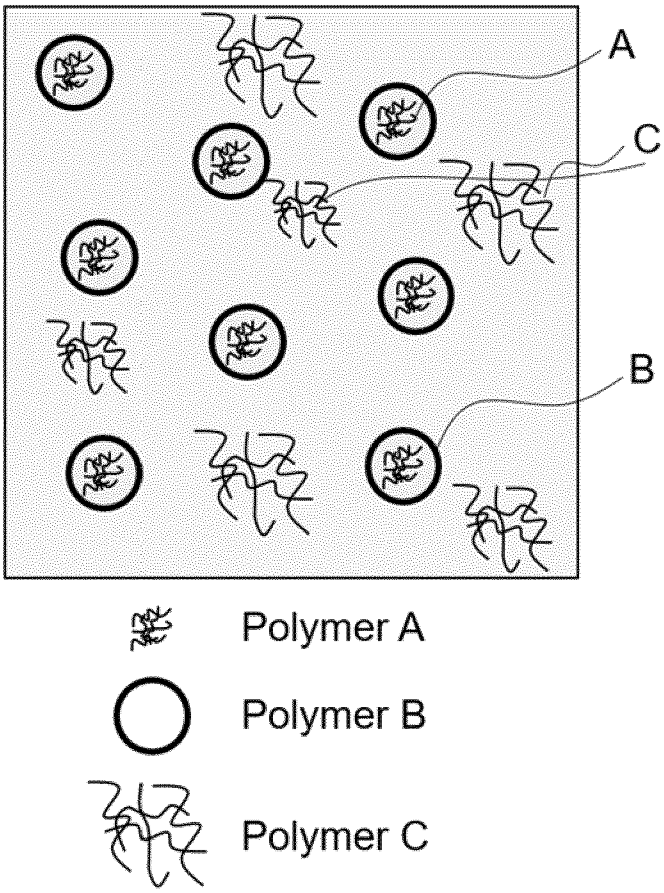

[Fig. 4]

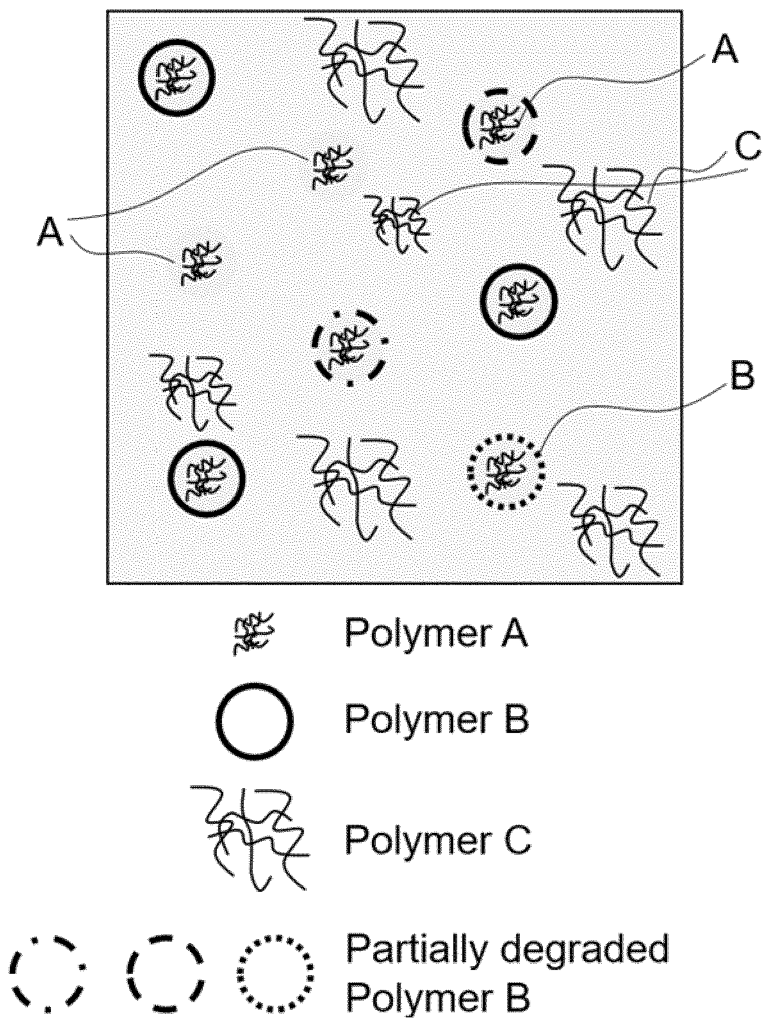
A
C
A
B
Polymer A
Polymer B
Polymer C
Partially degraded
Polymer B

[Fig. 5]

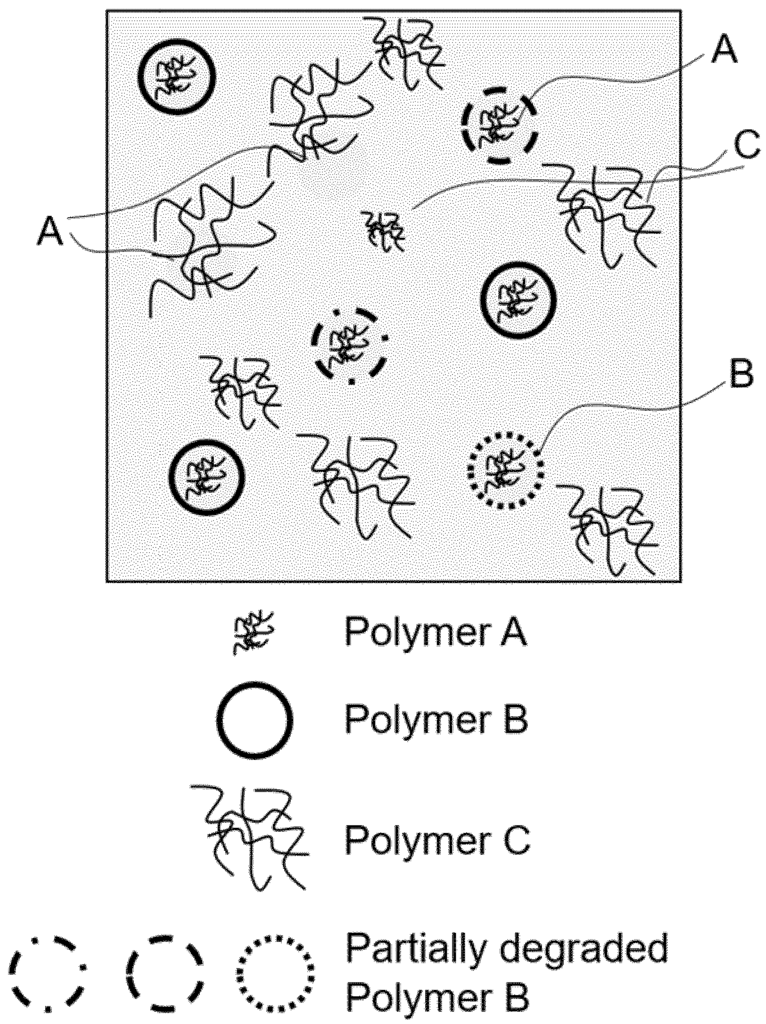
A
C
A
B
Polymer A
Polymer B
Polymer C
Partially degraded
Polymer B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019115619 A
- FR 3075219
- US 20020012645 A
- EP 1764078 A1
- US 10759746 B
- FR 2979821
- FR 2766838
- FR 2744131
- EP 0759966 A